# EUROPÄISCHE PATENTSCHRIFT

(11) **EP 4 034 053 B1**
(45) Veröffentlichungstag und Bekanntmachung des Hinweises auf die Patenterteilung: **16.07.2025**
(21) Anmeldenummer: 20775189.2
(22) Anmeldetag: 01.09.2020
(51) Int. Cl.: A61F 2/958, A61M 25/10, A61M 25/00

(54) **ABKNICKSICHERER BALLONKATHETER**
KINK-PROOF BALLOON CATHETER
CATHÉTER À BALLONNET ANTI-TORSION

(30) Priorität: 25.09.2019 DE 102019125858
(43) Veröffentlichungstag der Anmeldung: 03.08.2022
(73) Patentinhaber: Ruebben, Alexander, 98000 Monaco (MC)
(72) Erfinder: Ruebben, Alexander, 98000 Monaco (MC)
(74) Vertreter: Schneiders & Behrendt Bochum
(86) Internationale Anmeldenummer: PCT/EP2020/074287
(87) Internationale Veröffentlichungsnummer: WO 2021/058236

(56) Entgegenhaltungen:
- DE-A1- 102013 021 998
- US-A1- 2010 016 937
- US-B2- 8 088 121

## Beschreibung

Die Erfindung betrifft einen Ballonkatheter mit einem sich in Längsrichtung erstreckenden Schaft, der zumindest einen ersten und einen zweiten Abschnitt aufweist, wobei der erste Abschnitt distal des zweiten Abschnitts angeordnet und flexibler ist als der zweite Abschnitt.

Der Einsatz von Ballonkathetern gehört heutzutage zum Standard im klinischen Alltag. Ihre Verwendung im Rahmen von intravaskulären Interventionen bezieht sich meist auf die Aufweitung verengter Gefäßstellen, entweder mit Hilfe des Ballonkatheters an sich oder in Kombination mit weiteren Medizinprodukten wie beispielsweise ballonexpandierbaren Stents. Bei der perkutanen transluminalen Angioplastie wird ein Ballonkatheter über einen Führungsdraht und einen Führungskatheter an den Ort der Stenose gebracht und durch Zufuhr eines Fluids unter Druck (ca. 4 bis 12 bar) expandiert. Im Bereich der Stenose vorhandene Ablagerungen werden in die Gefäßwandung gedrückt. Zusätzlich kann zwecks dauerhafter Offenhaltung des Blutgefäßes ein Stent (Gefäßendoprothese) gesetzt werden. Um das erneute Auftreten einer Stenose durch ein gefäßverengendes Überwuchern der erweiterten Stelle zu verhindern, können auch medikamentenbeschichtete Ballonkatheter eingesetzt werden, die bei der Expansion einen Wirkstoff wie Paclitaxel an der Stelle der Gefäßverengung abgeben. Nach erfolgter Behandlung und dem anschließenden Zusammenfalten des Ballons wird der Ballonkatheter aus dem Gefäßsystem zurückgezogen und entfernt. 1 Bar entspricht 100000 Pa.

Als Zugangsstelle für Ballonkatheter wird häufig die Arteria femoralis im Bereich der Leiste gewählt. Die Arteria femoralis verläuft zumindest partiell relativ oberflächlich und ist daher für den behandelnden Arzt gut zugänglich, sowohl für durchzuführende Behandlungen im koronaren Bereich, aber auch für andere Bereiche des Gefäßsystems wie Gehirn oder Extremitäten. Darüber hinaus lassen sich über die Arteria femoralis (Leistenarterie) verhältnismäßig großlumige Katheter mit großen Außendurchmessern einbringen.

Eine Problematik beim Einführen von Ballonkathetern besteht in dem Zielkonflikt, einerseits den Ballonkatheter über verhältnismäßig lange Distanzen vorschiebbar auszubilden, andererseits jedoch einen ausreichend dünnen und flexiblen Ballonkatheter zur Verfügung zu haben, sodass dieser auch in englumige Blutgefäße eingebracht werden kann. Wichtig ist darüber hinaus, den Ballonkatheter möglichst abknicksicher auszubilden, damit der durch den behandelnden Arzt von proximal erfolgende Vorschub des Ballonkatheters bis zur distalen Spitze des Ballonkatheters übertragen wird, ohne dass der Vorschub des Ballonkatheters durch Abknicken zwischen der Zugangsstelle und der Zielposition gefährdet wird.

Hinsichtlich einer guten Vorschiebbarkeit und Abknicksicherheit wäre grundsätzlich ein verhältnismäßig steifer Ballonkatheter von Vorteil, hinsichtlich der Einführbarkeit in englumige, stark gewundene Blutgefäße hingegen eine möglichst große Flexibilität. Um diesen sich eigentlich widersprechenden Eigenschaften gerecht zu werden, sind Ballonkatheter häufig im proximalen Bereich verhältnismäßig steif und im distalen Bereich verhältnismäßig flexibel ausgebildet. Beispielsweise kann ein proximaler Abschnitt aus einem Metall wie z. B. Edelstahl gefertigt sein, während weiter distal gelegene Abschnitte aus einem Kunststoffmaterial, häufig einem Polyamid, bestehen können.

In diesem Zusammenhang sind auch Ballonkatheter bekannt, die in einem distalen Abschnitt aus einem besonders flexiblen Material, in einem mittleren Abschnitt aus einem Material mittlerer Flexibilität und in einem proximalen Abschnitt aus einem vergleichsweise wenig flexiblen Material aufgebaut sind. Grundsätzlich wird hiermit das Kriterium der von proximal nach distal zunehmenden Flexibilität erfüllt, die Übergänge zwischen den einzelnen Abschnitten sind jedoch potentielle Schwachstellen, an denen ein Um- bzw.

Abknicken des Ballonkatheters beim Vorschub in Richtung distal erfolgen kann. Das Dokument DE-A-102013021998 offenbart einen Ballonkatheter gemäß Präambel des Anspruchs 1.

Es stellt sich daher die Aufgabe, einen Ballonkatheter zur Verfügung zu stellen, der diese Problematik überwindet und insbesondere keine Stellen aufweist, an denen eine erhöhte Gefahr des Abknickens vorliegt.

Diese Aufgabe wird erfindungsgemäß gelöst durch einen Ballonkatheter mit einem sich in Längsrichtung erstreckenden Schaft, der zumindest einen ersten und einen zweiten Abschnitt aufweist, wobei der erste Abschnitt distal des zweiten Abschnitts angeordnet und flexibler als der zweite Abschnitt ist, wobei der Schaft einen ersten und einen zweiten schlauchförmigen Tubus aufweist und am distalen Ende des ersten Tubus ein Ballon angeordnet ist, der durch Druckbeaufschlagung mit einem durch den ersten Tubus geleiteten Fluid expandierbar ist, und der zweite Tubus der Aufnahme eines Führungsdrahts dient, distal des Ballons endet und am distalen Ende eine Öffnung aufweist, wobei sowohl der erste als auch der zweite Tubus entlang des ersten und zweiten Abschnitts verlaufen und sowohl der erste als auch der zweite Tubus im ersten Abschnitt flexibler sind als im zweiten Abschnitt, wobei zwischen dem ersten und dem zweiten Abschnitt ein Übergangsabschnitt angeordnet ist, in dem
- der erste Tubus die gleichen Materialeigenschaften aufweist wie im ersten Abschnitt und der zweite Tubus die gleichen Materialeigenschaften wie im zweiten Abschnitt oder
- der erste Tubus die gleichen Materialeigenschaften aufweist wie im zweiten Abschnitt und der zweite Tubus die gleichen Materialeigenschaften wie im ersten Abschnitt.

Die Erfindung beruht somit auf dem Gedanken, zwischen dem ersten und dem zweiten Abschnitt einen Übergangsabschnitt zu schaffen, der zum einen insgesamt eine Flexibilität aufweist, die zwischen der des ersten und der des zweiten Abschnitts liegt. Dies wird dadurch erreicht, dass im Übergangsabschnitt die Materialeigenschaften des ersten Tubus sich von denen des zweiten Tubus unterscheiden. Zudem wird entweder für den ersten oder für den zweiten Tubus Material verwendet, das in seinen Eigenschaften dem im ersten oder zweiten Abschnitt entspricht. Mit anderen Worten überlappen sich die Materialeigenschaften. Zwar gilt sowohl für den ersten als auch für den zweiten Tubus, dass sie im ersten Abschnitt flexibler sind als im zweiten Abschnitt, der Übergang zwischen dem ersten, besonders flexiblen Abschnitt und dem zweiten, weniger flexiblen Abschnitt erfolgt jedoch nicht an einer einzelnen Position, sondern letztlich an zwei Positionen, nämlich dem distalen und dem proximalen Ende des Übergangsabschnitts. Hierdurch wird die Gefahr, dass am Übergang vom ersten zum zweiten Abschnitt ein Abknicken erfolgt, erheblich reduziert.

Im Übergangsabschnitt werden die Materialeigenschaften von erstem und zweitem Tubus überlappend ausgebildet. Entweder entsprechen die Materialeigenschaften des ersten Tubus im Übergangsabschnitt denen im ersten Abschnitt, während der zweite Tubus die Materialeigenschaften des zweiten Abschnitts aufweist, oder umgekehrt. Dadurch wird erreicht, dass sich am distalen und am proximalen Ende des Übergangsabschnitts die Materialeigenschaften von einem der beiden Tuben nicht ändern, hier also eine Materialkonstanz vorliegt, die bewirkt, dass ein Abknicken erheblich unwahrscheinlicher wird. Sowohl am distalen als auch am proximalen Ende des Übergangsabschnitts ändern sich lediglich die Eigenschaften eines der beiden Tuben, jedoch nicht die Eigenschaften des jeweils anderen Tubus.

Wie herkömmliche Ballonkatheter auch verfügt der erfindungsgemäße Ballonkatheter zumindest über zwei Tuben, nämlich einen ersten Tubus, der der Fluidzufuhr zwecks Expansion des Ballons dient, und einem zweiten Tubus, der für die Aufnahme eines Führungsdrahtes vorgesehen ist. Entsprechend ist am distalen Ende des ersten Tubus der Ballon angeordnet. Wenn der Ballon des Ballonkatheters seine Zielposition erreicht hat, wird das Fluid durch den ersten Tubus in den Ballon eingebracht, um diesen zu expandieren und beispielsweise Ablagerungen an den Gefäßen in die Gefäßwand einzudrücken oder auch einen Stent aufzuweiten. Anschließend wird das Fluid durch den ersten Tubus wieder abgezogen, woraufhin der Ballon deflatiert, sodass der Ballonkatheter anschließend insgesamt in Richtung proximal aus dem Blutgefäßsystem herausgezogen werden kann. Der Begriff Tubus wird in diesem Zusammenhang im Sinne einer Röhre oder eines Schlauchs verwendet, der sich zumindest teilweise durch den Ballonkatheter in Längsrichtung erstreckt und ein durch das Innere des Tubus verlaufendes Lumen aufweist. Dabei kann der Tubus die Form eines Hohlzylinders mit kreisförmigem oder elliptischem Querschnitt aufweisen, dies ist jedoch nicht unbedingt erforderlich. Denkbar sind im Querschnitt betrachtet auch nahezu beliebige andere Formen. Ein kreisförmiger oder auch elliptischer Querschnitt weist allerdings den Vorteil auf, dass ein Tubus gut durch den anderen Tubus hindurch geführt werden kann, wobei in der Regel der zweite Tubus durch den ersten Tubus gelegt wird.

Der zweite Tubus dient der Aufnahme des Führungsdrahts. Im Gegensatz zum ersten Tubus ist dieser distal nicht geschlossen, sondern endet distal des Ballons und weist am distalen Ende eine Öffnung auf. In der Regel geht man so vor, dass zunächst der Führungsdraht an die Zielposition gebracht und anschließend der Ballonkatheter über den Führungsdraht an die Zielposition vorgeschoben wird, wobei der Führungsdraht durch den zweiten Tubus gleitet.

Im Zusammenhang mit dem zweiten Tubus sind im Wesentlichen zwei unterschiedliche Systeme bekannt, nämlich Over-The-Wire (OTW)- und Rapid Exchange (Rx)-Ballonkatheter. Der erfindungsgemäße Ballonkatheter kann sowohl als OTW- als auch als Rx-Ballonkatheter vorliegen. Während sich bei einem OTW-Katheter das Lumen für den Führungsdraht über die gesamte Länge des Katheters von proximal nach distal erstreckt, verfügt der Rx-Katheter über eine separate Zuführöffnung für den Führungsdraht (Rx-Port), an der der Führungsdraht deutlich distal des proximalen Endes des Katheters aus dem Katheter austritt. Entsprechend laufen die Tuben bzw. die sich durch die Tuben erstreckenden Lumen für die Fluidzufuhr und den Führungsdraht im Falle eines OTW-Ballonkatheters parallel oder konzentrisch zueinander vom proximalen Ende des Katheters bis zum Ballon, während dies bei einem Rx-Katheter nur zwischen Rx-Port und Ballon der Fall ist. Der Abschnitt zwischen Rx-Port und proximalem Ende hingegen weist nur einen Tubus für die Fluidzufuhr auf.

Um die Materialeigenschaften im ersten und zweiten Abschnitt sowie im Übergangsabschnitt in der gewünschten Art und Weise einzustellen, können insbesondere im ersten Abschnitt der erste und der zweite Tubus aus einem ersten Material gefertigt sein und im zweiten Abschnitt der erste und der zweite Tubus aus einem zweiten Material, wobei das erste Material flexibler ist als das zweite Material und im Übergangsabschnitt der erste Tubus aus einem anderen Material gefertigt ist als der zweite Tubus. Für einen der beiden Tuben wird somit das Material zwischen erstem Abschnitt und Übergangsabschnitt konstant gehalten, für den anderen Tubus zwischen Übergangsabschnitt und zweitem Abschnitt. Auf diese Weise wird der beschriebene überlappende Übergang zwischen den Materialeigenschaften von erstem und zweitem Tubus herbeigeführt.

Als erstes, besonders flexibles oder weiches Material, bieten sich thermoplastische Elastomere, beispielsweise Polyetherblockamide (PEBA) an. Hierbei handelt es sich um ein thermoplastisches Elastomer, das durch Polykondensation eines Carbonsäurepolyamids mit einem Polyether mit endständigen OH-Gruppen erhältlich ist. Insbesondere wird PEBA unter der Bezeichnung PEBAX^{®} von der Firma Arkema vertrieben. Als flexibles Material wird in diesem Zusammenhang ein Material verstanden, dass sich besonders gut den äußeren Gegebenheiten anpasst und auch feinen Verästelungen des Gefäßsystems folgen kann, wobei weich und flexibel im Rahmen dieser Anmeldung synonym gebraucht werden.

Alternativ können auch andere Polyamide als erstes Material für den Ballon verwendet werden, beispielsweise solche, wie sie unter der Bezeichnung Grilamid^{®} von der Firma EMS-GRIVORY vertrieben werden. Besonders bevorzugt ist die Verwendung eines Polyamids 12 (PA 12, Grilamid^{®} L), einem durch die Polykondensation von Laurinlactam erhältlichen Polyamid. Weitere verwendbare Polyamide sind Polyamid 10.10 (PA 10.10, Grilamid^{®} 1S), ein durch Polykondensation von Decandiamin und Sebacinsäure erhältliches Polyamid, Polyamid 6.10 (PA 6.10, Grilamid^{®} 2S), ein durch Polykondensation von Hexamethylendiamin und Sebacinsäure erhältliches Polyamid oder Polyamid 6.12 (PA 6.12, Grilamid^{®} 2D), ein durch Polykondensation von Hexamethylendiamin und Dodecandisäure erhältliches Polyamid.

Als zweites Material mittlerer Flexibilität kann z. B. ein Polyamid wie Nylon (Polyhexamethylenadipinsäureamid) verwendet werden. Insbesondere kann für den ersten Abschnitt ein Material mit einer Shore D-Härte im Bereich von ca. 25 - 72 verwendet werden. Für den zweiten Abschnitt bietet sich hingegen die Verwendung von Materialien mit einer Shore D-Härte im Bereich von 80 - 85 an. Die genauen Eigenschaften der Polymere können durch Zugabe von Zusatzstoffen eingestellt werden.

Das Material, aus dem der Ballon selbst aufgebaut ist, kann mit dem Material des ersten Tubus im ersten Abschnitt übereinstimmen oder sich von diesem unterscheiden. Beispielsweise kann der Ballon selbst auch aus Nylon (Polyhexamethylenadipinsäureamid) gefertigt sein, das sich als Material für Ballons bewährt hat, selbst wenn der erste Tubus im ersten Abschnitt aus einem flexibleren Material wie einem Polyetherblockamid aufgebaut ist. Andere für den Ballon verwendbare Materialien sind Polyurethan, Polyolefincopolymere, Polyethylen oder Silikone.

Unter Ballon im Sinne der Erfindung wird das durch Zufuhr eines Fluids expandierbare Element eines Ballonkatheters verstanden, unabhängig davon, welche Form das expandierbare Element aufweist oder aus welchem Material es besteht. Typischerweise hat der Ballon einen länglichen Aufbau. Das Fluid kann gasförmig oder flüssig sein. Als Fluid kann bspw. mit Kontrastmittel versetztes Wasser oder eine mit Kontrastmittel versetzte Kochsalzlösung dienen. Der Nominaldruck zur Expansion des Ballons kann z. B. 4 bis 12 bar betragen, bevorzugt 6 bis 8 bar. Bei diesem Druck erreicht der Ballon seinen nominalen Durchmesser im expandierten Zustand. Die Dimensionen des Ballons können je nach Anwendungsbereich stark differieren, der Durchmesser im expandierten Zustand kann beispielsweise zwischen ca. 1 und ca. 50 mm liegen, die Länge zwischen ca. 5 und ca. 300 mm. Ggf. können die Maße hiervon aber auch abweichen, beispielsweise bei Einsatz des Ballons/Ballonkatheters in der Urologie oder Veterinärmedizin.

Typischerweise liegt der deflatierte Ballon des Ballonkatheters in einem gefalteten Zustand vor. Je nach Größe des Ballons können dabei unterschiedlich viele Falten ausgebildet sein, die anschließend um die Achse des Katheters in gleicher Richtung aufgewunden werden. Hierdurch wird eine deutliche Reduktion des Durchmessers erreicht.

Alternativ oder zusätzlich zur Variation des Materials selbst können sich auch im Übergangsabschnitt die Materialstärken des ersten und des zweiten Tubus unterscheiden. Beispielsweise kann zwar für den ersten und den zweiten Abschnitt das gleiche Material verwendet werden, jedoch in unterschiedlicher Stärke. In diesem Fall sind sowohl der erste als auch der zweite Tubus im ersten Abschnitt aus dem Material mit vergleichsweise geringer Materialstärke gefertigt, während der erste und der zweite Tubus im zweiten Abschnitt aus dem gleichen (oder auch einem anderen) Material, jedoch mit höherer Materialstärke aufgebaut sind. Im Übergangsabschnitt wird wiederum ein überlappender Übergang in der Weise erzeugt, dass die Materialeigenschaften des einen Tubus mit denen im ersten Abschnitt übereinstimmen und die Materialeigenschaften des anderen Tubus mit denen im zweiten Abschnitt. Somit hat entweder der erste Tubus im ersten Abschnitt und im Übergangsabschnitt die gleiche Materialstärke, während der zweite Tubus im Übergangsabschnitt und im zweiten Abschnitt die gleiche Materialstärke aufweist, oder der erste Tubus weist im Übergangsabschnitt und im zweiten Abschnitt die gleiche Materialstärke auf, während der zweite Tubus im ersten Abschnitt und im Übergangsabschnitt die gleiche Materialstärke hat. Dabei müssen die Materialstärken von erstem und zweitem Tubus in einem bestimmten Abschnitt nicht unbedingt übereinstimmen; beispielsweise kann auch der zweite Tubus im ersten Abschnitt eine geringere Materialstärke aufweisen als der erste Tubus. Wichtig ist jedoch, dass nicht für den ersten und den zweiten Tubus der Übergang der Materialstärken an einer einzigen Stelle entlang der Längsachse des Ballonkatheters erfolgt, sondern in der beschriebenen Art und Weise versetzt zueinander.

Bei Herbeiführung eines Übergangsabschnitts durch Variation der Materialstärken kann als Material für den ersten und den zweiten Tubus z. B. Nylon (Polyhexamethylenadipinsäureamid) eingesetzt werden, wobei, anders als bei der Variation des Materials selbst, die abweichenden Eigenschaften durch Wahl einer höheren oder niedrigeren Materialstärke herbeigeführt werden.

Unter proximal wird im Rahmen der Erfindung in Richtung Körperäußeres, d. h. zum behandelnden Arzt hin verstanden, unter distal die entgegengesetzte Richtung, d. h. in Richtung des zu behandelnden Blutgefäßes. Unter radial wird die Ebene senkrecht zur Längsachse des Ballonkatheters verstanden.

Der erste und der zweite Tubus können in den Bereichen, wo beide Tuben vorliegen, parallel zueinander verlaufen, bevorzugt ist jedoch, dass der zweite Tubus sich zumindest teilweise durch den ersten Tubus erstreckt. Entsprechend liegt ein konzentrischer Verlauf von erstem und zweitem Tubus vor. Normalerweise liegt dabei der zweite Tubus innen, d. h. durch den inneren zweiten Tubus des Ballonkatheters wird der Führungsdraht geführt, während der erste Tubus den zweiten Tubus radial umgibt.

Proximal des zweiten Abschnitts ist vorzugsweise ein weiterer, proximaler Abschnitt angeordnet. Dieser weist typischerweise eine geringere Flexibilität auf als der erste oder der zweite Abschnitt, macht dafür jedoch einen erheblichen Teil der Gesamtlänge des Ballonkatheters aus. Möglich ist es auch, zwischen dem zweiten Abschnitt und dem proximalen Abschnitt weitere Abschnitt vorzusehen. Hinsichtlich des proximalen Abschnitts steht weniger die Flexibilität als vielmehr die Vorschiebbarkeit und Abknicksicherheit im Vordergrund. Entsprechend kann der proximale Abschnitt beispielsweise aus einem Metall, insbesondere aus Edelstahl gefertigt sein. Möglich ist jedoch ebenso das Vorsehen eines proximalen Abschnitts aus einem Polymer, wobei dieses Polymer typischerweise steifer ist als die für den ersten und zweiten Abschnitt sowie den Übergangsabschnitt verwendeten Polymere.

Am proximalen Ende des Ballonkatheters, sich anschließend an den Schaft, ist zumeist ein sog. Katheterhub vorgesehen, d. h. ein Anschlussstück für die Vorrichtung zur Fluidzufuhr und Druckbeaufschlagung. Die Verbindung kann z. B. eine herkömmliche Luer- bzw. Luer-Lock-Verbindung sein. Insbesondere ist es sinnvoll, zwei Luer-Lock-Anschlüsse, typischerweise weibliche Anschlüsse vorzusehen, von denen einer dem Anschluss des ersten Lumens an einen Ballondilatator und ein weiterer der Einführung des Führungsdrahts in den Ballonkatheter dient. Die Anschlüsse können beispielsweise aus einem Polycarbonat gefertigt sein. Der durch den Ballonkatheter verlaufende Führungsdraht kann an seinem proximalen Ende durch einen Torquer gehalten werden, was die Handhabung des in der Regel sehr dünnen Führungsdrahts erleichtert.

Damit der Übergangsabschnitt die gewünschte Rolle als Sicherung gegenüber einem unerwünschten Abknicken des Ballonkatheters erfüllen kann, sollte dieser eine Länge von ≥ 3 cm, vorzugsweise 3 bis 10 cm aufweisen. Besonders bevorzugt ist eine Länge des Übergangsabschnitts von 5 bis 7 cm. Ein zu kurz gewählter Übergangsabschnitt würde unter Umständen den gewünschten Abknickschutz nicht mehr herbeiführen, ein zu lang gewählter Übergangsabschnitt hingegen wäre unter Umständen für die Einführung in englumige Blutgefäße nicht mehr geeignet.

Als Länge des ersten Abschnitts haben sich 3 bis 20 cm, insbesondere 5 bis 15 cm, als Länge des zweiten Abschnitts 5 bis 35 cm, insbesondere 20 bis 30 cm bewährt. Unter Länge des ersten Abschnitts wird in diesem Zusammenhang die Länge von der distalen Spitze des Ballonkatheters bis zum Beginn des Übergangsabschnitts verstanden. Die Gesamtlänge des Ballonkatheters beträgt häufig mehr als 1 m, sodass eine Einführung in der Leistenregion möglich ist und der Ballonkatheter an unterschiedlichste Stellen im Blutgefäßsystem vorgeschoben werden kann. Häufig weist allein der proximale Abschnitt eine Länge ≥ 1 m auf.

Ein typischer Außendurchmesser des ersten Tubus im ersten Abschnitt beträgt 0,8 - 1,0 mm, insbesondere ca. 0,9 mm. Der Innendurchmesser liegt typischerweise in einem Bereich von 0,7 - 0,8 mm. Der Außendurchmesser des zweiten Tubus kann beispielsweise 0,5 - 0,6 mm, der Innendurchmesser des zweiten Tubus 0,4 - 0,5 mm betragen. Dies gilt insbesondere, wenn sich der zweite Tubus durch den ersten Tubus erstreckt. Im zweiten Abschnitt kann der Außendurchmesser des ersten Tubus geringfügig höher liegen als im ersten Abschnitt und beispielsweise 0,9 - 1,1 mm betragen. Der Innendurchmesser des ersten Tubus liegt im zweiten Abschnitt zumeist bei 0,8 - 0,9 mm, während der zweite Tubus Dimensionen aufweist, die weitgehend denen im ersten Abschnitt entsprechen.

Um zu verhindern, dass es in der Folge einer zunächst erfolgreich durchgeführten Angioplastie zu einer erneuten Verengung in dem behandelten Gefäßabschnitt kommt, können Ballons mit einer Wirkstoffbeschichtung eingesetzt werden. Eine Restenose ist meist auf eine Zellproliferation im entsprechenden Gefäßabschnitt zurückzuführen, d. h. Zellen des Blutgefäßes wachsen in das Gefäßlumen hinein und sorgen wiederum für eine Behinderung des Blutflusses. Um dies zu verhindern, werden zunehmend mit proliferationshemmend wirkenden Medikamenten beschichtete Ballonkatheter eingesetzt. Entsprechende Medikamente wirken meist insbesondere auf die *Smooth Muscle Cells* (SMC) und sollen so eine durch ein übermäßiges Wachstum dieser Zellen bedingte Restenose verhindern. Das Medikament befindet sich auf der Außenseite des Ballons und wird während der Ballondilatation vom Ballon auf beziehungsweise in die Gefäßinnenwand übertragen.

Bei dem verwendeten Wirkstoff handelt es sich insbesondere um ein Arzneimittel bzw. Medikament, bevorzugt um ein Arzneimittel, das proliferationshemmend wirkt und das gefäßverengende Überwuchern der durch den Ballon erweiterten Stelle verhindert. Ebenso kann es sich um einen hormonartigen oder regulierenden Wirkstoff handeln, der an bestimmten Zellen organspezifische Wirkungen oder Regulationsfunktionen beeinflussen kann. Insbesondere kann der Wirkstoff ausgewählt sein aus: Tretinoin, Orphanrezeptoragonisten, Elafinderivate, Corticosteroide, Steroidhormone, Paclitaxel, Rapamycin, Tacrolimus, hydrophobe Proteine sowie zellproliferationsverändernde Substanzen. Es ist auch möglich, Gemische dieser Wirkstoffe zu verwenden. Darüber hinaus können auch Derivate der genannten Wirkstoffe verwendbar sein, wobei unter Derivaten insbesondere Salze, Ester und Amide verstanden werden. Als Steroidhormone können beispielsweise Methylprednisolon, Dexamethason oder Östradiol verwendet werden. Besonders bevorzugt ist die Verwendung von Paclitaxel, Rapamycin oder Tacrolimus bzw. entsprechenden Derivaten.

Allgemein ist der Begriff Wirkstoff jedoch weit zu verstehen, d. h. es kann sich grundsätzlich um beliebige Beschichtungen auf dem Ballon des Ballonkatheters handeln, mit denen am Zielort eine bestimmte Wirkung erzielt werden soll. Diese Wirkung kann bei Einbringung in Blutgefäße insbesondere in der Zellproliferationshemmung bestehen. Auf anderen Gebieten der Medizin kann die gewünschte Wirkung jedoch eine andere sein, so etwa im Bereich der Urologie bei Blasenkathetern, wo die Beschichtung insbesondere der Hemmung der Bakterienbesiedlung dienen soll. Hier kann als Wirkstoff beispielsweise Heparin eingesetzt werden.

Die Beschichtung der Oberfläche des Ballons mit dem Wirkstoff erfolgt typischerweise dadurch, dass die Oberfläche des Ballons mit einer Lösung des Wirkstoffs in Kontakt gebracht wird. Dies kann insbesondere durch Eintauchen des Ballons in die Lösung erfolgen. Das Eintauchen dauert dabei in der Regel max. 1 min, typischerweise 10 bis 30 s. Der Ballon sollte nach dem Eintauchen mit einer Geschwindigkeit von bis zu 10 mm/s aus der ersten Lösung herausgezogen werden. Noch günstiger ist es, wenn das Herausziehen mit einer Geschwindigkeit von weniger als 5 mm/s, vorzugsweise mit einer Geschwindigkeit zwischen 0,5 mm/s und 2 mm/s erfolgt. Durch das langsame Herausziehen wird ein langsames Trocknen der Oberfläche erreicht.

Vor der Beschichtung des Ballons ist es sinnvoll, die Oberfläche des Ballons zu reinigen. Dies kann beispielsweise mit einem entsprechenden Lösungsmittel erfolgen, beispielsweise dem auch für die Aufbringung des Wirkstoffs verwendeten Lösungsmittel.

Die Lösung kann hinsichtlich des Wirkstoffs gesättigt sein, dies ist jedoch nicht unbedingt erforderlich. Als Lösungsmittel können beispielsweise Methylenchlorid, Chloroform, Alkohol, insbesondere Ethanol, Methanol oder Isopropanol, Aceton, Diethylether, flüssige Kohlenwasserstoffe, wie zum Beispiel Pentan, Hexan, Heptan, Cyclohexan oder Octan, Toluol, Tetrahydrofuran (THF) oder Essigester verwendet werden. Möglich ist auch die Verwendung von Lösungsmittelgemischen. Vorzugsweise handelt es sich um eine Lösung des Wirkstoffs in Methylenchlorid.

Alternativ zur Beschichtung durch Eintauchen kann diese auch auf andere Weise erfolgen, bspw. durch Besprühen.

Die erfindungsgemäßen Ballonkatheter können in Blutgefäßen eingesetzt werden, insbesondere im Bereich der Angioplastie. In diesem Fall ist der Zielort des Ballons ein Blutgefäß, wobei Blutgefäße in unterschiedlichen Bereichen in Frage kommen, insbesondere koronar, intrakraniell und peripher. Es ist jedoch auch möglich, Ballonkatheter in anderen medizinischen Bereichen einzusetzen. Eine Einsatzmöglichkeit liegt in der Urologie, wo Ballonkatheter als Blasenkatheter in die Harnblase eingesetzt werden. Über den Ballon wird der Katheter fixiert. Hier kann der Ballon z. B. mit einer Beschichtung versehen sein, die bakterieller Besiedlung und Inkrustation vorbeugt, beispielsweise mit Heparin.

In der Pneumologie können Ballonkatheter zur Aufweitung oder zum Verschluss eines Bronchus eingesetzt werden. Auch in der Gynäkologie können Ballonkatheter zum Einsatz kommen. Im Bereich der Orthopädie können Ballonkatheter zur Behandlung von Wirbelbrüchen durch Wiederaufrichtung der Wirbel mittels Ballonexpansion eingesetzt werden (Ballon-Kyphoplastie). Der erfindungsgemäße Ballonkatheter ist grundsätzlich auf sämtlichen Gebieten der Medizin einsetzbar, in denen Ballonkatheter zum Einsatz kommen, wobei der Ballonkatheter besondere Bedeutung bei der Einführung in englumige Blutgefäße hat.

Darüber hinaus kann der erfindungsgemäße Ballonkatheter nicht nur der Beseitigung von Stenosen und der lokalen Wirkstoffeinbringung, sondern zusätzlich der Platzierung eines Stents (Endoprothese) im Körperlumen dienen. Stents sind röhrenartige Stützstrukturen, die in einem Körperlumen, bspw. einem Blutgefäß implantiert werden, um dieses dauerhaft offen zu halten. Derartige Stents können selbstexpandierend sein oder mit Hilfe eines Ballons expandiert werden. Hierzu wird der Stent auf dem Ballon aufgecrimpt und mit Hilfe des Ballonkatheters in das Körperlumen eingebracht. An der vorgesehenen Stelle wird sodann der Ballon durch Zufuhr eines Fluids expandiert, wodurch sich auch der Stent weitet und im Körperlumen verankert wird. Gleichzeitig wird bei Verwendung des erfindungsgemäßen Ballons der Wirkstoff an die Wandung des Körperlumens abgegeben. Schließlich wird der Ballon wieder zusammengezogen und aus dem Körperlumen entfernt, während der Stent im Körperlumen verbleibt.

Entlang des Ballonkatheters können an verschiedenen Positionen röntgendichte Markierungen angebracht sein, die der Visualisierung des Katheters im Röntgenbild dienen. Insbesondere kann es sich dabei um Markierungen aus Platin oder einer Platinlegierung wie Platin-Iridium handeln.

Die Erfindung wird anhand der beigefügten Figuren beispielhaft näher erläutert. Es zeigen:
- Fig. 1:: Einen erfindungsgemäßen Ballonkatheter in der Seitenansicht;
- Fig. 2: den distalen Teil des Schafts des Ballonkatheters aus Fig. 1 im Längsschnitt.

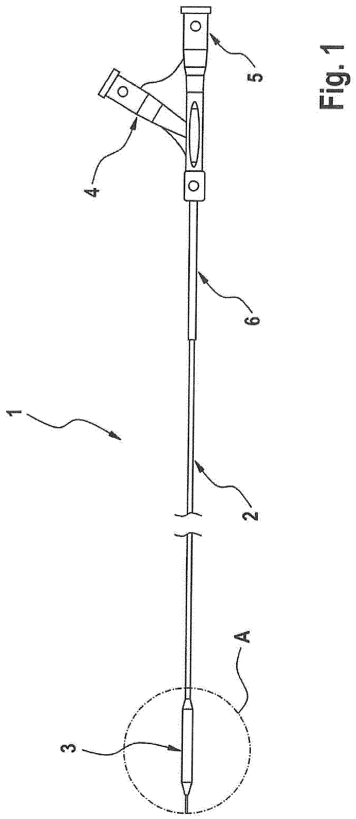

In Fig. 1 ist der erfindungsgemäße Ballonkatheter 1 in der Seitenansicht dargestellt, wobei in der hier gewählten Darstellung rechts proximal und links distal bedeutet. Der Ballonkatheter 1 weist einen sich in Längsrichtung erstreckenden Schaft 2 auf, dessen Außendurchmesser im hier nur verkürzt dargestellten proximalen Abschnitt 6 größer ist als weiter distal. Der Abschnitt des Schafts 2, in dem der Ballon 3 angeordnet ist, ist mit dem Buchstaben A gekennzeichnet. Durch den Schaft 2 verlaufen (hier nicht dargestellt) ein erstes Lumen für die Fluidzufuhr sowie ein zweites Lumen zur Aufnahme des Führungsdrahts, wobei die beiden Lumen jeweils durch einen Tubus gebildet werden.

Proximal schließen sich an den proximalen Abschnitt 6 des Schafts 2 zwei Katheterhubs in Form von Luer-Lock-Verbindungen 4, 5 an, wobei die Verbindung 4 der Fluidzufuhr in das erste Lumen mittels eines Ballondilatators und die Verbindung 5 der Einführung des Führungsdrahts in das zweite Lumen dienen.

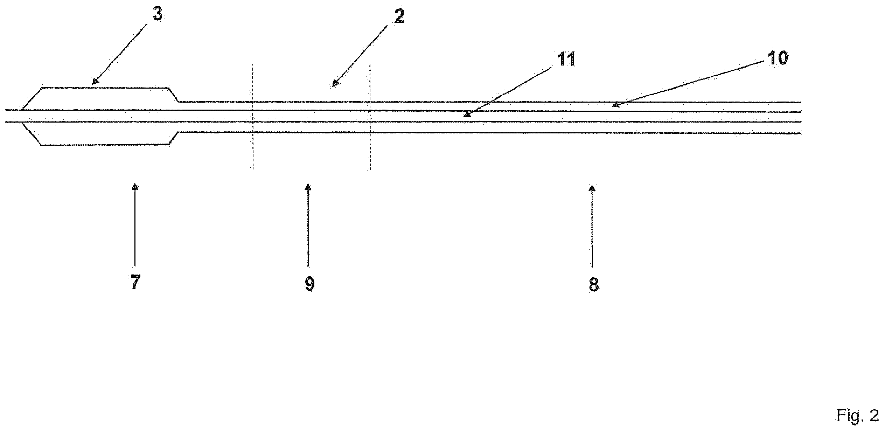

In Fig. 2 ist lediglich der distale Teil des Schafts 2 dargestellt. Ein erster Tubus 10 ist so gestaltet, dass er den zweiten Tubus 11 aufnehmen kann, d. h. der zweite Tubus 11 verläuft in Längsrichtung durch den ersten Tubus 10. Der erste Tubus 10 steht an seinem distalen Ende mit dem Ballon 3 in Verbindung, welcher durch Fluidzufuhr durch den ersten Tubus 10 expandiert werden kann. Der zweite Tubus 11 hingegen verfügt am distalen Ende über eine Öffnung und dient der Aufnahme des hier nicht dargestellten Führungsdrahts.

Der hier dargestellte Teil des Schafts 2 (ohne den proximalen Abschnitt 6 aus Fig. 1) weist einen ersten Abschnitt 7 und einen zweiten Abschnitt 8 auf, wobei der erste Abschnitt 7 distal des zweiten Abschnitts 8 angeordnet ist. Erster und zweiter Abschnitt 7, 8 grenzen jedoch nicht direkt aneinander, stattdessen befindet sich ein Übergangsabschnitt 9 zwischen dem ersten Abschnitt 7 und dem zweiten Abschnitt 8. Im Übergangsabschnitt 9 ist der erste Tubus 10 im hier gewählten Beispiel aus dem gleichen Material hergestellt wie im ersten Abschnitt 7, beispielsweise aus PEBAX^{®}. Der zweite Tubus 11 hingegen ist im Übergangsabschnitt 9 aus dem gleichen Material hergestellt ist wie im zweiten Abschnitt 8, beispielsweise aus Nylon. Der Übergang vom steiferen zum flexibleren Material erfolgt somit für den ersten und den zweiten Tubus 10, 11 an unterschiedlichen Stellen, in einem Fall beim Übergang vom zweiten Abschnitt 8 zum Übergangsabschnitt 9, im anderen Fall beim Übergang vom Übergangsabschnitt 9 zum ersten Abschnitt 7. Die Eigenschaften des jeweils anderen Tubus bleiben an den entsprechenden Stellen hingegen unverändert, sodass die Gefahr des Abknickens deutlich reduziert ist. Im vorliegenden Beispiel hat der erste Tubus 10 (einschließlich Ballonbereich) am distalen Ende eine Länge von 15 cm für das weichere Material und weiter proximal eine Länge von 25 cm für das weniger weiche Material. Der zweite Tubus 11 hingegen hat am distalen Ende eine Länge von 10 cm für das weichere Material und weiter proximal eine Länge von 30 cm für das weniger weiche Material. Somit hat der erste Abschnitt 7 eine Länge von 10 cm, der Übergangsabschnitt 9 von 5 cm und der zweite Abschnitt 8 eine Länge von 25 cm.

## Patentansprüche

1. Ballonkatheter mit einem sich in Längsrichtung erstreckenden Schaft (2), der zumindest einen ersten und einen zweiten Abschnitt (7, 8) aufweist, wobei der erste Abschnitt (7) distal des zweiten Abschnitts (8) angeordnet und flexibler als der zweite Abschnitt (8) ist, wobei der Schaft (2) einen ersten und einen zweiten schlauchförmigen Tubus (10, 11) aufweist und am distalen Ende des ersten Tubus (10) ein Ballon (3) angeordnet ist, der durch Druckbeaufschlagung mit einem durch den ersten Tubus (10) geleiteten Fluid expandierbar ist, und der zweite Tubus (11) der Aufnahme eines Führungsdrahts dient, distal des Ballons (3) endet und am distalen Ende eine Öffnung aufweist, wobei sowohl der erste als auch der zweite Tubus (10, 11) entlang des ersten und zweiten Abschnitts (7, 8) verlaufen und sowohl der erste als auch der zweite Tubus (10, 11) im ersten Abschnitt (7) flexibler sind als im zweiten Abschnitt (8), **dadurch gekennzeichnet,**
**dass** zwischen dem ersten und dem zweiten Abschnitt (7, 8) ein Übergangsabschnitt (9) angeordnet ist, in dem
- der erste Tubus (10) die gleichen Materialeigenschaften aufweist wie im ersten Abschnitt (7) und der zweite Tubus (11) die gleichen Materialeigenschaften wie im zweiten Abschnitt (8) oder
- der erste Tubus (10) die gleichen Materialeigenschaften aufweist wie im zweiten Abschnitt (8) und der zweite Tubus (11) die gleichen Materialeigenschaften wie im ersten Abschnitt (7).

2. Ballonkatheter nach Anspruch 1, **dadurch gekennzeichnet, dass** im ersten Abschnitt (7) der erste und der zweite Tubus (10, 11) aus einem ersten Material gefertigt sind und im zweiten Abschnitt (8) der erste und der zweite Tubus (10, 11) aus einem zweiten Material gefertigt sind, wobei das erste Material flexibler ist als das zweite Material und im Übergangsabschnitt (9) der erste Tubus (10) aus einem anderen Material gefertigt ist als der zweite Tubus (11).

3. Ballonkatheter nach Anspruch 2, **dadurch gekennzeichnet, dass** das erste Material ein thermoplastisches Elastomer ist.

4. Ballonkatheter nach Anspruch 2 oder 3, **dadurch gekennzeichnet, dass** das zweite Material ein Polyamid ist.

5. Ballonkatheter nach einem der Ansprüche 1 bis 4, **dadurch gekennzeichnet, dass** sich im Übergangsabschnitt (9) die Materialstärken des ersten und des zweiten Tubus (10, 11) unterscheiden.

6. Ballonkatheter nach einem der Ansprüche 1 bis 5, **dadurch gekennzeichnet, dass** sich der zweite Tubus (11) zumindest teilweise durch den ersten Tubus (10) erstreckt.

7. Ballonkatheter nach einem der Ansprüche 1 bis 6, **dadurch gekennzeichnet, dass** proximal des zweiten Abschnitts (8) ein proximaler Abschnitt (6) angeordnet ist.

8. Ballonkatheter nach Anspruch 7, **dadurch gekennzeichnet, dass** der Schaft (2) im proximalen Abschnitt (6) zumindest teilweise aus Metall gefertigt ist.

9. Ballonkatheter nach Anspruch 8, **dadurch gekennzeichnet, dass** der Schaft (2) im proximalen Abschnitt (6) zumindest teilweise aus Edelstahl gefertigt ist.

10. Ballonkatheter nach einem der Ansprüche 1 bis 9, **dadurch gekennzeichnet, dass** der Übergangsabschnitt (9) eine Länge von ≥ 3 cm, vorzugsweise 3 bis 10 cm aufweist.

11. Ballonkatheter nach einem der Ansprüche 1 bis 10, **dadurch gekennzeichnet, dass** die Länge des ersten Abschnitts (7) 3 - 20 cm, insbesondere 5 - 15 cm beträgt.

12. Ballonkatheter nach einem der Ansprüche 1 bis 11, **dadurch gekennzeichnet, dass** die Länge des zweiten Abschnitts (8) 5 - 35 cm, insbesondere 20 - 30 cm beträgt.

13. Ballonkatheter nach einem der Ansprüche 1 bis 12, **dadurch gekennzeichnet, dass** der Ballon (3) mit einem oder mehreren Wirkstoffen beschichtet ist.

14. Ballonkatheter nach Anspruch 13, **dadurch gekennzeichnet, dass** der verwendete Wirkstoff ausgewählt ist aus der Gruppe: Tretinoin, Orphanrezeptoragonisten, Elafinderivate, Corticosteroide, Steroidhormone, Paclitaxel, Rapamycin, Tacrolimus, hydrophobe Proteine, Heparin und/oder hormonartige oder zellproliferationsverändernde Substanzen.

## Claims

1. Balloon catheter comprising a shaft (2) extending in the longitudinal direction, which has at least a first and a second section (7, 8), with the first section (7) being arranged distal to the second section (8) and being more flexible than the second section (8), with the shaft (2) being provided with a first and a second hose-like tube (10, 11) and a balloon (3) being arranged at the distal end of the first tube (10), said balloon (3) being expandable by pressurization with a fluid led through said first tube (10), and said second tube (11) serving for accommodating a guidewire and terminating distally of said balloon (3) and being provided with an opening at said distal end, wherein both said first and second tubes (10, 11) extend along said first and second sections (7, 8), and both said first and second tubes (10, 11) are more flexible in said first section (7) than in said second section (8), **characterized in that**
a transition section (9) is arranged between said first and second sections (7, 8), in which
- the first tube (10) has the same material properties as in the first section (7) and the second tube (11) has the same material properties as in the second section (8) or
- the first tube (10) has the same material properties as in the second section (8) and the second tube (11) has the same material properties as in the first section (7).

2. Balloon catheter according to claim 1, **characterized in that** in the first section (7) the first and the second tube (10, 11) are made of a first material and in the second section (8) the first and the second tube (10, 11) are made of a second material, with the first material being more flexible than the second material and in the transition section (9) the first tube (10) being made of another material than that of the second tube (11).

3. Balloon catheter according to claim 2, **characterized in that** the first material is a thermoplastic elastomer.

4. Balloon catheter according to claim 2 or 3, **characterized in that** the second material is a polyamide.

5. Balloon catheter according to any one of claims 1 to 4, **characterized in that** the material thicknesses of the first and second tubes (10, 11) differ in the transition section (9).

6. Balloon catheter according to any one of claims 1 to 5, **characterized in that** the second tube (11) extends at least partially through the first tube (10).

7. Balloon catheter according to any one of claims 1 to 6, **characterized in that** a proximal section (6) is arranged proximal to the second section (8).

8. Balloon catheter according to claim 7, **characterized in that** the shaft (2) in the proximal section (6) is at least partially made of metal.

9. Balloon catheter according to claim 8, **characterized in that** the shaft (2) in the proximal section (6) is at least partially made of stainless steel.

10. Balloon catheter according to any one of claims 1 to 9, **characterized in that** the transition section (9) has a length of ≥ 3 cm, preferably ranging between 3 and 10 cm.

11. Balloon catheter according to any one of claims 1 to 10, **characterized in that** the length of the first section (7) amounts to 3 to 20 cm, in particular ranges between 5 and 15 cm.

12. Balloon catheter according to any one of claims 1 to 11, **characterized in that** the length of the second section (8) amounts to 5 to 35 cm, in particular ranges between 20 and 30 cm.

13. Balloon catheter according to any one of claims 1 to 12, **characterized in that** the balloon (3) is coated with one or more active substances.

14. Balloon catheter according to claim 13, **characterized in that** the active agent used is selected from the following group: Tretinoin, orphan receptor agonists, elafin derivatives, corticosteroids, steroid hormones, paclitaxel, rapamycin, tacrolimus, hydrophobic proteins, heparin and/or hormone-like or cell proliferation-modifying substances.

## Revendications

1. Cathéter à ballonnet comprenant une tige (2) s'étendant dans le sens longitudinal, qui présente au moins une première et une deuxième section (7, 8), la première section (7) étant disposée de manière distale par rapport à la deuxième partie (8) et étant plus flexible que la deuxième section (8), la tige (2) comportant un premier et un deuxième tube tubulaire (10, 11) et un ballon (3) étant disposé à l'extrémité distale du premier tube (10), lequel ballon peut être gonflé par mise sous pression avec un fluide acheminé à travers le premier tube (10), et le deuxième tube (11) servant à recevoir un fil-guide, se termine à distance du ballon (3) et comporte une ouverture à son extrémité distale, le premier et le deuxième tube (10, 11) s'étendant le long de la première et de la deuxième section (7, 8) et le premier et le deuxième tube (10, 11) sont plus flexibles dans la première section (7) que dans la deuxième section (8), **caractérisé en ce**
**qu'**entre la première et la deuxième section (7, 8) est disposée une section de transition (9) dans laquelle
- le premier tube (10) présente les mêmes propriétés matérielles que dans la première section (7) et le deuxième tube (11) présente les mêmes propriétés matérielles que dans la deuxième section (8) ou,
- le premier tube (10) présente les mêmes propriétés matérielles que dans la deuxième section (8) et le deuxième tube (11) présente les mêmes propriétés matérielles que dans la première section (7).

2. Cathéter à ballonnet selon la revendication 1, **caractérisé en ce que**, dans la première section (7), le premier et le deuxième tube (10, 11) sont fabriqués dans un premier matériau et, dans la deuxième section (8), le premier et le deuxième tube (10, 11) sont fabriqués dans un deuxième matériau, le premier matériau étant plus flexible que le deuxième matériau et, dans la section de transition (9), le premier tube (10) étant fabriqué dans un matériau différent de celui du deuxième tube (11).

3. Cathéter à ballonnet selon la revendication 2, **caractérisé en ce que** le premier matériau est un élastomère thermoplastique.

4. Cathéter à ballonnet selon la revendication 2 ou 3, **caractérisé en ce que** le deuxième matériau est un polyamide.

5. Cathéter à ballonnet selon l'une des revendications 1 à 4, **caractérisé en ce que** l'épaisseur des matériaux du premier et du deuxième tube (10, 11) diffère dans la section de transition (9).

6. Cathéter à ballonnet selon l'une des revendications 1 à 5, **caractérisé en ce que** le deuxième tube (11) s'étend au moins partiellement à travers le premier tube (10).

7. Cathéter à ballonnet selon l'une des revendications 1 à 6, **caractérisé en ce qu'**une section proximale (6) est disposée à proximité de la deuxième section (8).

8. Cathéter à ballonnet selon la revendication 7, **caractérisé en ce que** la tige (2) est au moins partiellement fabriquée en métal dans la partie proximale (6).

9. Cathéter à ballonnet selon la revendication 8, **caractérisé en ce que** la tige (2) est au moins partiellement fabriquée en acier inoxydable dans la partie proximale (6).

10. Cathéter à ballonnet selon l'une des revendications 1 à 9, **caractérisé en ce que** la section de transition (9) présente une longueur de ≥ 3 cm, de préférence de 3 à 10 cm.

11. Cathéter à ballonnet selon l'une des revendications 1 à 10, **caractérisé en ce que** la longueur de la première section (7) est comprise entre 3 et 20 cm, en particulier entre 5 et 15 cm.

12. Cathéter à ballonnet selon l'une des revendications 1 à 11, **caractérisé en ce que** la longueur de la deuxième section (8) est comprise entre 5 et 35 cm, en particulier entre 20 et 30 cm.

13. Cathéter à ballonnet selon l'une des revendications 1 à 12, **caractérisé en ce que** le ballon (3) est recouvert d'un ou plusieurs principes actifs.

14. Cathéter à ballonnet selon la revendication 13, **caractérisé en ce que** le principe actif utilisé est choisi parmi le groupe comprenant : la trétinoïne, les agonistes des récepteurs orphelins, les dérivés de l'élafine, les corticostéroïdes, les hormones stéroïdes, le paclitaxel, la rapamycine, le tacrolimus, les protéines hydrophobes, l'héparine et/ou les substances hormonales ou modifiant la prolifération cellulaire.
